# EUROPÄISCHE PATENTSCHRIFT

(11) **EP 0 648 484 B1**
(45) Veröffentlichungstag und Bekanntmachung des Hinweises auf die Patenterteilung: **11.11.1998**
(21) Anmeldenummer: 94115245.6
(22) Anmeldetag: 28.09.1994
(51) Int. Cl.: A61K 6/083, C08K 9/02, C09C 1/28, C09C 3/06, C03C 17/00

(54) **Verfahren zur Herstellung eines mit Retention versehenen anorganischen Füllstoffs und seine Verwendung**
Process for the preparation of an inorganic filter having retentions and its use
Procédé de préparation d'une matière de change ayant des rétentions et son utilisation

(30) Priorität: 07.10.1993 DE 4334211
(43) Veröffentlichungstag der Anmeldung: 19.04.1995
(73) Patentinhaber: Heraeus Kulzer GmbH, 63450 Hanau (DE)
(72) Erfinder: Hengchang, Xu, Prof., Dental Hospital, Hai Dian District, Beijing (CH); Nagel, Joachim, D-61381 Friedrichsdorf (DE)
(74) Vertreter: Kühn, Hans-Christian

(56) Entgegenhaltungen:
- EP-A- 0 271 236
- EP-A- 0 324 242
- EP-A- 0 345 581
- DE-A- 4 105 319
- DE-C- 2 711 014
- US-A- 3 656 921
- US-A- 4 336 301
- Römpp, 9. Auflage, 1990, 2. Band, S. 1454 und 3. Band, S. 2425

## Beschreibung

Die Erfindung betrifft ein Verfahren zur Herstellung eines feinteiligen anorganischen Füllstoffs in Form eines Verbundfüllstoffs aus Makrofüllstoff-Teilchen und Mikrofüllstoff-Teilchen und die Verwendung des nach dem Verfahren hergestellten Füllstoffs in Kunststoffmaterialien. Der Füllstoff wird bevorzugt in Dentalmaterialien und Überzugsmaterial auf Kunststoff-Basis eingesetzt.

Auf dem Gebiet der durch Polymerisation aushärtenden Zahnfüllungsmaterialien bedeutete es einen großen Fortschritt, als Rafael L. Bowen anstelle des bis dahin darin verwendeten Methylmethacrylats langkettige monomere Dimethacrylate - Reaktionsprodukte des Bisphenol A und seiner Derivate mit Glycidylmethacrylat, besonders das sogenannte Bis-GMA, - und zur Verstärkung der Kunststoff-Matrix feines Quarzglas-Pulver einführte (US 3 066 112 A).

Ein weiteres Beispiel für ein Dentalmaterial, das neben organischen Monomeren einen feinteiligen anorganischen Füllstoff enthält, wird in US 3 539 533 A beschrieben. Das polymerisierbare Bindemittel ist dabei eine Mischung aus Bis-GMA, Bisphenol A-dimethacrylat, verdünnendem Monomer, besonders Triethylenglykoldimethacrylat, und gegebenenfalls Methacrylsäure in geringer Menge, das zusammen mit etwa 65 - 75 Gewichts-% des anorganischen Füllstoffs, zum Beispiel Siliciumdioxid, Glas, Aluminiumoxid oder Quarz, verwendet wird. Der anorganische Füllstoff kann eine Teilchengröße von etwa 2 - 85 Mikrometer besitzen; zur Verbesserung des Verbundes Kunststoff/Füllstoff wird er mit einem Silan, zum Beispiel 3-Methacryloyloxypropyltrimethoxysilan, vorbehandelt.

Aus anorganische Füllstoffe der unterschiedlichsten chemischen Zusammensetzung - meist aus Glas, Keramik oder Glaskeramik und zur Verbesserung der Füllstoff/Kunststoff-Haftung mit einem Silanisierungsmittel behandelt - enthaltenden Dentalmaterialien (Composites) lassen sich Zahnfüllungen, Kronen, künstliche Zähne und dergleichen mit guten mechanischen Festigkeitseigenschaften herstellen.

Der Einsatz mikrofeiner anorganischer Füllstoffe mit mittleren Teilchengrößen von 0,01 - 0,4 Mikrometer führte dann zu auch in ästhetischer Hinsicht verbesserten dentalen Kunststoff-Produkten mit Hochglanzpolierbarkeit und der Transparenz natürlicher Zähne ähnlicher Transparenz (DE 24 03 211 C3).

Einen weiteren Schritt in der Entwicklung von Dentalmaterialien auf Kunststoff-Basis stellen die sogenannten Hybrid-Materialien dar, die sowohl mikrofeine Füllstoffe als auch konventionelle Füllstoffe (Makrofüllstoffe) enthalten. Ein solches Dentalmaterial ist zum Beispiel aus DE 24 05 578 C3 bekannt. Es enthält 30 - 80 Gewichts-% einer Mischung aus durch Flammhydrolyse hergestellter amorpher Kieselsäure (pyrogenes Siliciumdioxid) mit einer maximalen Teilchengröße von 0,07 Mikrometer und feinteiligem Glas, bevorzugt Borsilicatglas, Barium- oder Lanthanoxid enthaltendes Glas oder Lithiumaluminiumsilicatglas, mit einer Teilchengröße bis zu 5 Mikrometer.

Das in DE 34 03 040 C2 beschriebene dentale Füllungsmaterial enthält 60 - 90 Gewichts-% eines Füllstoff-Gemisches aus 5 - 20 Gewichts-% eines röntgenopaken Füllstoffs mit einer Korngrößenverteilung zwischen 0,5 und 40 Mikrometer, 20 - 35 Gewichts-% eines röntgenopaken Füllstoffs mit einer Korngrößenverteilung zwischen 0,2 und 15 Mikrometer und 45 - 75 Gewichts-% eines Siliciumdioxid-Mikrofüllstoffs mit einer Korngrößenverteilung zwischen 5 und 150 Nanometer.

Ein weiteres Beispiel für ein Hybrid-Material ist die in EP 382 033 A2 beschriebene Dentalmasse, die neben polymerisierbaren Acrylaten oder Methacrylaten und einem Katalysator für die Photopolymerisation (Photoaktivator) 5 - 80 Gewichts-% eines silanisierten Glases oder einer silanisierten Glaskeramik mit einer mittleren Teilchengröße zwischen 0,1 und 10 Mikrometer und 2 - 10 Gewichts-% eines oberflächenbehandelten Mikrofüllers enthält. Die zur Verstärkung von Dentalmaterialien auf Kunststoff-Basis eingesetzten anorganischen Füllstoffe besitzen meist eine mit einem Silan, zum Beispiel 3-Methacryloyloxypropyltrimethoxysilan, behandelte Oberfläche, die die Verträglichkeit mit den organischen Bestandteilen verbessert (DE 34 03 040 C2) und eine chemische Haftung zwischen dem Füllstoff und der Kunststoff-Matrix bewirkt. Eine weitere Verbesserung des Füllstoff/Kunststoff-Verbundes kann erreicht werden, wenn zusätzlich zu der chemischen Haftung die Möglichkeit einer physikalischen Haftung besteht. Eine physikalische Haftung läßt sich beispielsweise nach einem Vorschlag in US 4 215 033 A durch die Verwendung eines durch Ätzen eines Zweiphasen-Glases erhaltenen semiporösen Füllstoffs schaffen.

Aus US-A-3 656 921 ist es bekannt, Titandioxid-haltige Glasfritte-Teilchen auf etwa Sintertemperatur zu erhitzen, um eine Keimbildung und Rekristallisation des Titandioxids zu bewirken und dadurch als undurchsichtige Pigmente zu verwendende Glasfritten zu erhalten. Damit die Glasfritte-Teilchen während des Erhitzens nicht miteinander oder mit der Behälterwandung verkleben, werden die Glasfritte-Teilchen vorher mit einem ihre Oberfläche mindestens teilweise bedeckenden Überzug aus vorzugsweise Titandioxid versehen. Vor oder während des Aufbringens des Überzugs können die Glasfritte-Teilchen mit einem organischen Bindemittel behandelt werden. Die Teilchengröße der Glasfritte-Teilchen beträgt 0,03 - 0,25 inch (762 - 6350 Mikrometer), die des Titandioxid-Überzugs 0,05 - 5 Mikrometer.

US-A-4 336 301 betrifft einen Füllstoff für thermoplastische Polymere aus glimmerartigem Schichtsilicat mit darauf befindlichen kleinen Vorsprüngen aus einem Zusatzstoff mit durchschnittlicher Teilchengröße von 1 - 50 Mikrometer. Der Zusatzstoff, der aus Glas oder Epoxyharz bestehen kann und einen unterhalb der Zersetzungstemperatur des Schichtsilicats liegenden Schmelzpunkt aufweist, benetzt in schmelzflüssigem Zustand die Oberfläche des Schichtsilicats. Der Füllstoff, der durch Erhitzen von Mischungen des Schichtsilicats mit dem Zusatzstoff auf eine Temperatur oberhalb des Schmelzpunktes des Zusatzstoffes und Kühlen hergestellt werden kann, bewirkt eine Verbesserung von Biegemodul, absoluter Zugfestigkeit und Bruchdehnung der damit verstärkten thermoplastischen Polymeren.

Wirken abrasive Kräfte auf Erzeugnisse aus mit feinteiligen anorganischen Füllstoffen verstärkten Kunststoffen ein, so wird zunächst - ohne eine Beschädigung der Füllstoff-Teilchen selbst - die weichere Kunststoff-Matrix von der Oberfläche her abgetragen. Bei fortschreitender Abrasion der Kunststoff-Matrix verlieren die an der Oberfläche befindlichen Füllstoff-Teilchen den Halt in der Kunststoff-Matrix und brechen heraus. So kann zum Beispiel bei aus Composites gelegten Zahnfüllungen der durch Abrasion beim Kauen und Zähneputzen verursachte Abbau so groß sein, daß die Zahnfüllungen vorzeitig erneuert werden müssen.

Der Erfindung liegt daher die Aufgabe zugrunde, ein Verfahren zur Herstellung eines feinteiligen anorganischen Füllstoffs für Kunststoffe, der bei abrasiver Beanspruchung der damit gefüllten Kunststoffe länger in der Kunststoffmatrix-Oberfläche verbleibt, zu finden. Die Füllstoff-Teilchen sollen sich - im Sinne einer gleichmäßigen Silanisierung - gut silanisieren lassen und gut von der Kunststoff-Matrix beziehungsweise den diese bildenden Monomeren benetzt werden. Der Füllstoff soll für den Einsatz in polymerisierbaren Dentalmaterialien und zur Verstärkung von Kunststoff-Materialien geeignet sein.

Das die Lösung der Aufgabe darstellende Verfahren ist erfindungsgemäß dadurch gekennzeichnet, daß zur Herstellung von jeweils aus einem Makrofüllstoff-Teilchen und einer Anzahl von dieses teilweise bedeckenden Mikrofüllstoff-Teilchen bestehenden Verbundfüllstoff-Teilchen mit einer mittleren Teilchengröße von 0,3 - 12 Mikrometer eine innige Mischung aus a) einen niedrigeren Schmelzpunkt als die Mikrofüllstoff-Teilchen aufweisenden Makrofüllstoff-Teilchen aus Glas mit einer mittleren Teilchengröße von 0,2 - 10,0 Mikrometer und b) Mikrofüllstoff-Teilchen aus Glas oder Keramik mit einer mittleren Teilchengröße von 0,01 - 1,5 Mikrometer hergestellt, die Mischung bei 650 - 1200 ° C gesintert und das Sinterprodukt nach Zugabe einer Flüssigkeit einer Ultraschallbehandlung unterworfen wird.

Gegebenenfalls kann das Sinterprodukt vor der Ultraschallbehandlung gemahlen werden.

Bevorzugt erfolgt das Verfahren mit Makrofüllstoff-Teilchen und Mikrofüllstoff-Teilchen, deren Schmelzpunkte sich um mindestens 40°C unterscheiden.

Besonders bewährt hat es sich, Makrofüllstoff-Teilchen mit einer mittleren Teilchengröße von 0,2 - 2,0 Mikrometer und Mikrofüllstoff-Teilchen mit einer mittleren Teilchengröße von 0,01 - 0,3 Mikrometer einzusetzen. Makrofüllstoff-Teilchen und Mikrofüllstoff-Teilchen liegen vorzugsweise im Gewichtsverhältnis von 1 : 1 - 12 vor.

Hinsichtlich ihrer Größe werden Makrofüllstoff- und Mikrofüllstoff-Teilchen so ausgewählt, daß das Verhältnis ihrer Durchmesser 5 - 20 : 1 beträgt.

Die Sintertemperatur hängt von der Art der Makrofüllstoff- und Mikrofüllstoff-Teilchen ab und liegt im allgemeinen bei 650 - 1200°C. Sie wird so gewählt, daß der Makrofüllstoff während des Sinterns erweicht beziehungsweise zu schmelzen beginnt und der Mikrofüllstoff im festen Zustand verbleibt.

Die für das erfindungsgemäße Verfahren einzusetzenden Materialien müssen unlöslich und - wenn der herzustellende Verbundfüllstoff für dentale Zwecke verwendet werden soll - bioverträglich sein. Die Makrofüllstoff-Teilchen bestehen vorzugsweise aus Borosilicat-Glas oder Aluminosilicat-Glas, wie Bariumborosilicat- oder Lithium- und Bariumaluminosilicat-Glas. Die Mikrofüllstoff-Teilchen bestehen ebenfalls aus Glas oder aus einer Keramik. Als Keramik eignet sich das Nitrid, Carbid oder Oxid eines der Elemente Silicium, Zirkonium, Aluminium oder Titan; besonders bewährt haben sich SiO₂, ZrO₂, Al₂O₃ und TiO₂.

Im folgenden werden die einzelnen Schritte des erfindungsgemäßen Verfahrens ausführlicher beschrieben.

Mischen: Makrofüllstoff-Teilchen einer mittleren Teilchengröße von 0,2 - 10,0 Mikrometer werden mit Mikrofüllstoff-Teilchen einer mittleren Teilchengröße von 0,01 - 1,5 Mikrometer im Gewichtsverhältnis von 1 : 1 - 12 in einer Kugelmühle oder in einem anderen zum Mischen von Feststoffen geeigneten Mischer unter Zusatz einer Flüssigkeit, wie Wasser/Ethanol, innig miteinander vermischt. Das genaue Gewichtsverhältnis von Makrofüllstoff zu Mikrofüllstoff hängt von dem Schmelzpunkt, der Teilchengröße und der Teilchengrößenverteilung der Füllstoffe ab und läßt sich durch Vorversuche bestimmen. Die Mischungszeit hängt von der Mahlgeschwindigkeit und der Füllstoff-Menge ab.

Sintern: Die innige Mischung wird nach Entfernen von darin enthaltener Flüssigkeit etwa 1 - 3 Stunden lang bei etwa 650 - 1200°C gesintert. Die genauen Sinterbedingungen hängen von den Eigenschaften und der Menge der Füllstoffe ab und lassen sich durch Vorversuche bestimmen.

Dispergieren: Das Sinterprodukt wird unter Zugabe einer Flüssigkeit, zum Beispiel Wasser/Ethanol, unter der Einwirkung von Ultraschallwellen gleichmäßig in der Flüssigkeit suspendiert. Sollte es erforderlich sein, so kann das Sinterprodukt vor der Ultraschallbehandlung gemahlen werden.

Isolieren: Eventuell in der Suspension vorhandene gröbere Teilchen setzen sich beim Stehenlassen der Suspension ab. Aus der durch Dekantieren von den gröberen Teilchen befreiten Suspension wird dann durch Zentrifugieren der feinteilige Verbundfüllstoff abgetrennt und getrocknet. Die Ausbeute beträgt 40 - 70 %; die mittlere Teilchengröße des Verbundfüllstoffs liegt bei 0,3 - 12,0 Mikrometer.

Silanisieren: Vor oder nach dem Zentrifugieren kann der Verbundfüllstoff in an sich bekannter Weise durch Behandlung mit einem Silan, zum Beispiel 3-Methacryloyloxypropyltrimethoxysilan, silanisiert werden.

Die Oberfläche der nach dem erfindungsgemäßen Verfahren hergestellten Verbundfüllstoff-Teilchen zeichnet sich durch eine retentionsreiche Mikrostruktur aus, gebildet durch die in charakteristischer Anordnung auf den Makrofüllstoff-Teilchen befindlichen Mikrofüllstoff-Teilchen. Die Verbundfüllstoff-Teilchen lassen sich gleichmäßig silanisieren und beim Vermischen mit Monomeren gut benetzen.

Aufgrund der retentionsreichen Mikrostruktur bleiben die Teilchen des Verbundfüllstoffs selbst dann noch in der Kunststoff-Matrix verankert, wenn sie zu zwei Dritteln ihres Volumens aus der Matrix herausragen.

Das hinsichtlich der Mikrostruktur der Verbundfüllstoff-Teilchen und ihrer Verankerung in der Kunststoff-Matrix Gesagte wird durch die Zeichung mit den Figuren 1, 2 und 3 veranschaulicht.

Die Figur 1 zeigt in schematischer Darstellung ein Verbundfüllstoff-Teilchen aus dem den Kern bildenden Makrofüllstoff-Teilchen 1 und den damit verbundenen Mikrofüllstoff-Teilchen 2.

Die Figuren 2 (a - e) und 3 (a - e) geben in schematischer Darstellung das Verhalten von Teilchen eines bekannten Füllstoffs 1 und von Teilchen des erfindungsgemäßen Verbundfüllstoffs 2 in der Kunststoff-Matrix 3 bei fortschreitendem Abbau der Kunststoff-Matrix infolge Abrasion wieder.

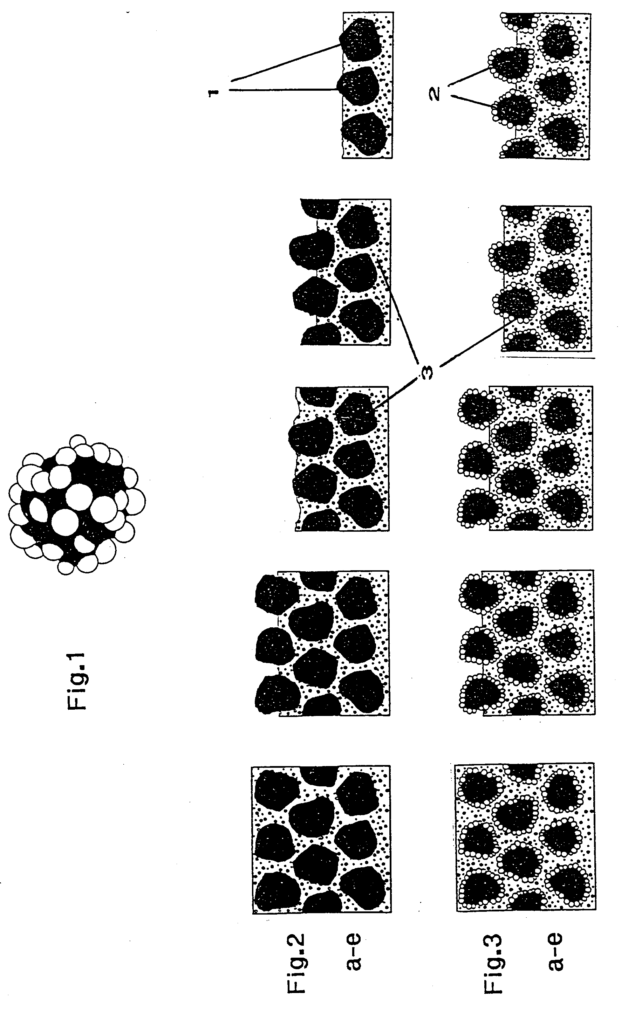

Zur näheren Erläuterung wird in den folgenden Beispielen die Herstellung eines feinteiligen Verbundfüllstoffs nach dem erfindungsgemäßen Verfahren und eine den Verbundfüllstoff enthaltende Formulierung zur Verwendung als durch Bestrahlung mit Licht polymerisierbares Dentalmaterial beschrieben. Die Abrasionsfestigkeit des ausgehärteten Dentalmaterials wird bestimmt und mit der handelsüblicher Dentalmaterialien nach Aushärtung verglichen.

### Beispiel 1

### Herstellung des Verbundfüllstoffs

10 g eines Bariumaluminiumsilicat-Glases mit einer mittleren Teilchengröße von 0,7 Mikrometer und 150 g mikrofeines Siliciumdioxid mit einer mittleren Teilchengröße von 0,04 Mikrometer (Aerosil OX50) werden in Gegenwart von 1500 ml eines Wasser/Ethanol-Gemisches (85 : 15) 80 Stunden lang in einer Kugelmühle innig miteinander vermischt. Anschließend wird die Mischung bei 105°C an Luft getrocknet und bei etwa 850°C 3 Stunden lang gesintert. Nach Abkühlen auf Raumtemperatur und Zugabe der zehnfachen Menge eines Wasser/Ethanol-Gemisches (85 : 15) wird das Sinterprodukt mit 100 ml einer 15%igen Lösung von 3-Methacryloyloxypropyltrimethoxysilan in Ethanol versetzt und dann das so erhaltene Gemisch 8 Stunden lang der Einwirkung von Ultraschallwellen ausgesetzt. Im Anschluß daran wird das Gemisch, damit sich gröbere Teilchen absetzen können, 3 Stunden lang stehengelassen, dann durch Dekantieren von den gröberen Teilchen befreit und durch Zentrifugieren mit 2800 Umdrehungen/Minute in Feststoff und Flüssigkeit getrennt. Nach dem Trocknen des Feststoffs bei 105°C an Luft liegen 110 g des gewünschten Verbundfüllstoffs mit einer mittleren Teilchengröße von 0,5 - 3 Mikrometer vor.

### Beispiel 2

### Herstellung eines Dentalmaterials

100 g eines polymerisierbaren Bindemittels werden durch Mischen von
54,24 g Bis-GMA,
44,33 g Triethylenglykoldimethacrylat,
0,99 g 2-(2-Hydroxy-5-methylphenyl)-benzotriazol,
0,16 g Campherchinon und
0,28 g 2-Diethylaminoethylmethacrylat
erhalten.

Durch Mischen von 30 g des polymerisierbaren Bindemittels, 68 g des in Beispiel 1 beschriebenen feinteiligen Verbundfüllstoffs und 2 g eines mikrofeinen Siliciumdioxids einer mittleren Teilchengröße von 0,04 Mikrometer (Aerosil OX50) wird ein besonders für den Seitenzahnbereich geeignetes Zahnfüllungsmaterial in Form einer homogenen Paste hergestellt.

Proben des Zahnfüllungsmaterials werden in Glasröhrchen (Innendurchmesser 10 mm, Höhe 6 mm) gegeben und 360 Sekunden lang mit einem Lichtgerät bestrahlt. Die Bestimmung der Abrasionsfestigkeit der so erhaltenen Prüfkörper erfolgt über die Messung des Volumenverlustes, wie in Journal of Oral Rehabilitation, 1990, Volumen 17, 107-115, beschrieben. Zum Vergleich werden in entsprechender Weise aus vier handelsüblichen Zahnfüllungsmaterialien vom Composite-Typ hergestellte polymere Prüfkörper untersucht. Der an den Prüfkörpern nach 2000 Cyclen gemessene Volumenverlust als Mittelwert von zehn Messungen, die Standardabweichung und die Varianz (Standardabweichung/Mittelwert) werden in der Tabelle angegeben.

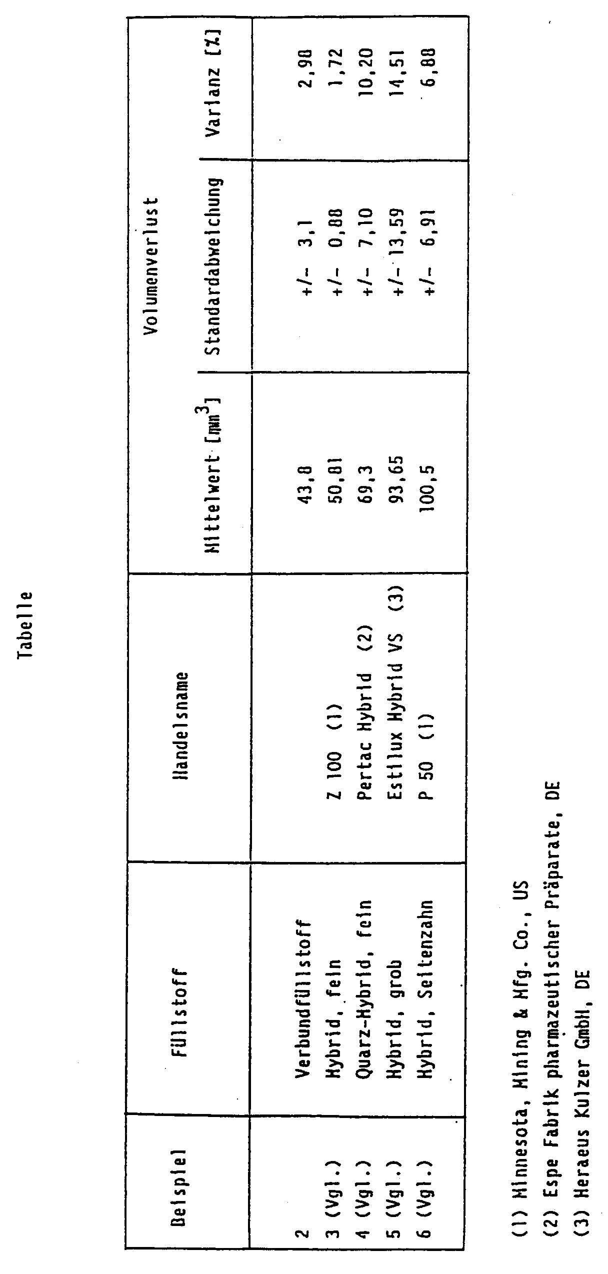

## Patentansprüche

1. Verfahren zur Herstellung eines feinteiligen anorganischen Füllstoffs in Form eines Verbundfüllstoffs aus Makrofüllstoff-Teilchen und Mikrofüllstoff-Teilchen, dadurch gekennzeichnet, daß zur Herstellung von jeweils aus einem Makrofüllstoff-Teilchen und einer Anzahl von dieses teilweise bedeckenden Mikrofüllstoff-Teilchen bestehenden Verbundfüllstoff-Teilchen mit einer mittleren Teilchengröße von 0,3 - 12 Mikrometer eine innige Mischung aus a) einen niedrigeren Schmelzpunkt als die Mikrofüllstoff-Teilchen aufweisenden Makrofüllstoff-Teilchen aus Glas mit einer mittleren Teilchengröße von 0,2 - 10,0 Mikrometer und b) Mikrofüllstoff-Teilchen aus Glas oder Keramik mit einer mittleren Teilchengröße von 0,01 - 1,5 Mikrometer hergestellt, die Mischung bei 650 - 1200 ° C gesintert und das Sinterprodukt nach Zugabe einer Flüssigkeit einer Ultraschallbehandlung unterworfen wird.

2. Verfahren nach Anspruch 1, dadurch gekennzeichnet, daß das Sinterprodukt vor der Ultraschallbehandlung gemahlen wird.

3. Verfahren nach Anspruch 1 oder 2, dadurch gekennzeichnet, daß Makrofüllstoff-Teilchen, die einen um mindestens 40°C niedrigeren Schmelzpunkt als die Mikrofüllstoff-Teilchen aufweisen, eingesetzt werden.

4. Verfahren nach einem der Ansprüche 1 bis 3, dadurch gekennzeichnet, daß Makrofüllstoff-Teilchen mit einer mittleren Teilchengröße von 0,2 - 2,0 Mikrometer eingesetzt werden.

5. Verfahren nach einem der Ansprüche 1 bis 4, dadurch gekennzeichnet, daß Mikrofüllstoff-Teilchen mit einer mittleren Teilchengröße von 0,01 - 0,3 Mikrometer eingesetzt werden.

6. Verfahren nach einem der Ansprüche 1 bis 5, dadurch gekennzeichnet, daß die Makrofüllstoff-Teilchen und die Mikrofüllstoff-Teilchen im Gewichtsverhältnis von 1 : 1 - 12 eingesetzt werden.

7. Verfahren nach einem der Ansprüche 1 bis 6, dadurch gekennzeichnet, daß Makrofüllstoff-Teilchen und Mikrofüllstoff-Teilchen, deren Durchmesser-Verhältnis 5 - 20 : 1 beträgt, eingesetzt werden.

8. Verfahren nach einem der Ansprüche 1 bis 7, dadurch gekennzeichnet, daß aus Borosilicat-Glas oder Aluminosilicat-Glas bestehende Makrofüllstoff-Teilchen eingesetzt werden.

9. Verfahren nach einem der Ansprüche 1 bis 8, dadurch gekennzeichnet, daß aus einem Nitrid, Carbid oder Oxid eines der Elemente Silicium, Zirkonium, Aluminium oder Titan bestehende Mikrofüllstoff-Teilchen eingesetzt werden.

10. Verfahren nach Anspruch 9, dadurch gekennzeichnet, daß aus SiO₂, ZrO₂, Al₂O₃ oder TiO₂ bestehende Mikrofüllstoff-Teilchen eingesetzt werden.

11. Verfahren nach einem der Ansprüche 1 bis 10, dadurch gekennzeichnet, daß der Verbundfüllstoff mit einem Silan behandelt wird.

12. Verfahren nach Anspruch 11, dadurch gekennzeichnet, daß der Verbundfüllstoff mit 3-Methacryloyloxypropyltrimethoxysilan behandelt wird.

13. Verwendung des nach dem Verfahren nach einem der Ansprüche 1 - 12 hergestellten feinteiligen Verbundfüllstoffs zur Herstellung von polymerisierbarem Dentalmaterial.

14. Verwendung des nach dem Verfahren nach einem der Ansprüche 1 - 12 hergestellten feinteiligen Verbundfüllstoffs in polymerisierbarem Material zur Herstellung von Überzügen.

15. Verwendung des nach dem Verfahren nach einem der Ansprüche 1 - 12 hergestellten feinteiligen Verbundfüllstoffs zur Verstärkung von natürlichen oder synthetischen organischen polymeren Materialien.

## Claims

1. Process for the preparation of a finely divided inorganic filler in the form of a composite filler comprising macrofiller particles and microfiller particles, characterized in that, for the preparation of composite filler particles which each consist of a macrofiller particle and a number of microfiller particles partly covering said macrofiller particle and which have a mean particle size of 0.3 - 12 micrometers, an intimate mixture of a) a glass macrofiller particle having a lower boiling point than the microfiller particles and having a mean particle size of 0.2 - 10.0 micrometers and b) glass or ceramic microfiller particles having a mean particle size of 0.01 - 1.5 micrometers is prepared, the mixture is sintered at 650 - 1200°C and the sintered product is subjected to an ultrasonic treatment after the addition of a liquid.

2. Process according to Claim 1, characterized in that the sintered product is milled before the ultrasonic treatment.

3. Process according to Claim 1 or 2, characterized in that macrofiller particles which have a melting point at least 40°C lower than the microfiller particles are used.

4. Process according to any of Claims 1 to 3, characterized in that macrofiller particles having a mean particle size of 0.2 - 2.0 micrometers are used.

5. Process according to any of Claims 1 to 4, characterized in that microfiller particles having a mean particle size of 0.01 - 0.3 micrometer are used.

6. Process according to any of Claims 1 to 5, characterized in that the macrofiller particles and the microfiller particles are used in a weight ratio of 1 : 1-12.

7. Process according to any of Claims 1 to 6, characterized in that macrofiller particles and microfiller particles whose diameter ratio is 5-20 : 1 are used.

8. Process according to any of Claims 1 to 7, characterized in that macrofiller particles consisting of borosilicate glass or aluminosilicate glass are used.

9. Process according to any of Claims 1 to 8, characterized in that microfiller particles consisting of a nitride, carbide or oxide of one of the elements silicon, zirconium, aluminium or titanium are used.

10. Process according to Claim 9, characterized in that microfiller particles consisting of SiO₂, ZrO₂, Al₂O₃ or TiO₂ are used.

11. Process according to any of Claims 1 to 10, characterized in that the composite filler is treated with a silane.

12. Process according to Claim 11, characterized in that the composite filler is treated with 3-methacryloyloxypropyltrimethoxysilane.

13. Use of the finely divided composite filler prepared by the process according to any of Claims 1 - 12 for the preparation of polymerizable dental material.

14. Use of the finely divided composite filler prepared by the process according to any of Claims 1 - 12 in polymerizable material for the production of coatings.

15. Use of the finely divided composite filler prepared by the process according to any of Claims 1 - 12 for reinforcing natural or synthetic organic polymeric materials.

## Revendications

1. Procédé de production d'une charge inorganique finement divisée sous forme d'une charge composite constituée de particules de charge macroscopique et de particules de charge microscopique, caractérisé en ce que, pour produire des particules de charge composite ayant une taille de particules moyenne de 0,3 à 12 µm, constituées chacune d'une particule de charge macroscopique et d'un certain nombre de particules de charge microscopique qui la recouvrent partiellement, on prépare un mélange intime a) de particules de charge macroscopique constituée de verre, ayant une taille de particules moyenne de 0,2 ― 10,0 µm et un point de fusion inférieur à celui des particules de charge microscopique, et b) de particules de charge microscopique constituée de verte ou de céramique et ayant une taille de particules moyenne de 0,01 à 1,5 µm, on fritte le mélange à 650 ― 1200°C et, après avoir ajouté un liquide, on soumet le produit fritté à un traitement aux ultrasons.

2. Procédé selon la revendication 1, caractérisé en ce que l'on broie le produit fritté avant le traitement aux ultrasons.

3. Procédé selon la revendication 1 ou 2, caractérisé en ce que l'on utilise des particules de charge macroscopique ayant un point de fusion inférieur d'au moins 40°C à celui des particules de charge microscopique.

4. Procédé selon l'une des revendications 1 à 3, caractérisé en ce que l'on utilise des particules de charge macroscopique ayant une taille de particules moyenne de 0,2 à 2,0 µm.

5. Procédé selon l'une des revendications 1 à 4, caractérisé en ce que l'on utilise des particules de charge microscopique ayant une taille de particules moyenne de 0,01 à 0,3 µm.

6. Procédé selon l'une des revendications 1 à 5, caractérisé en ce que l'on utilise les particules de charge macroscopique et les particules de charge microscopique en un rapport en masse de 1 : 1-12.

7. Procédé selon l'une des revendications 1 à 6, caractérisé en ce que l'on utilise des particules de charge macroscopique et des particules de charge microscopique dont le rapport des diamètres est de 5 ― 20 : 1.

8. Procédé selon l'une des revendications 1 à 7, caractérisé en ce que l'on utilise des particules de charge macroscopique constituées de verre de borosilicate ou de verre d'aluminosilicate.

9. Procédé selon l'une des revendications 1 à 8, caractérisé en ce que l'on utilise des particules de charge microscopique constituées d'un nitrure, d'un carbure ou d'un oxyde d'un des éléments silicium, zirconium, aluminium ou titane.

10. Procédé selon la revendication 9, caractérisé en ce que l'on utilise des particules de charge microscopique constituées de SiO₂, de ZrO₂, d'Al₂O₃ ou de TiO₂.

11. Procédé selon l'une des revendications 1 à 10, caractérisé en ce que l'on traite la charge composite avec un silane.

12. Procédé selon la revendication 11, caractérisé en ce que l'on traite la charge composite avec du 3-méthacryloyloxypropyltriméthoxysilane.

13. Utilisation de la charge composite finement divisée produite par le procédé selon l'une des revendications 1 à 12 pour la préparation d'un matériau dentaire polymérisable.

14. Utilisation de la charge composite finement divisée produite par le procédé selon l'une des revendications 1 à 12 dans un matériau polymérisable pour la production de revêtements.

15. Utilisation de la charge composite finement divisée produite par le procédé selon l'une des revendications 1 à 12 pour le renforcement de matériaux polymères organiques naturels ou synthétiques.
